# EUROPEAN PATENT SPECIFICATION

(11) **EP 1 966 193 B1**
(45) Date of publication and mention of the grant of the patent: **29.09.2010**
(21) Application number: 06841321.0
(22) Date of filing: 11.12.2006
(51) Int. Cl.: C07D 401/14, A61K 31/4439

(54) **NEW SALT AND POLYMORPH OF DPP-IV INHIBITOR**
NEUES SALZ UND POLYMORPH DES DPP-IV-HEMMERS
NOUVEAU SEL ET POLYMORPHE D UN INHIBITEUR DE DPP-IV

(30) Priority: 21.12.2005 EP 05112639
(43) Date of publication of application: 10.09.2008
(73) Proprietor: F. Hoffmann-La Roche AG, 4070 Basel (CH)
(72) Inventor: BUBENDORF, André, F-68510 Uffheim (FR); GRASSMANN, Olaf, 79540 Loerrach (DE); HUNZIKER, Daniel, CH-4313 Moehlin (CH); KUEHNE, Holger, 79540 Loerrach (DE); MOOG, Regina, 79104 Freiburg (DE); SCHWITTER, Urs, CH-4153 Reinach (CH)
(74) Representative: Salud, Carlos E.
(86) International application number: PCT/EP2006/069509
(87) International publication number: WO 2007/071576

(56) References cited:
- WO-A-03/037327

## Description

The compound (2S)-1-{[1,1-Dimethyl-3-(4-pyridin-3-yl-imidazol-1-yl)-propylamino]-acetyl}-pyrrolidine-2-carbonitrile is useful in the prophylaxis and/or treatment of diseases which are related with the enzyme dipeptidyl peptidase IV (EC.3.4.14.5, abbreviated in the following as DPP-IV). In WO 03/037327, the preparation of (2S)-1-{[1,1-Dimethyl-3-(4-pyridin-3-yl-imidazol-1-yl)-propylamino]-acetyl}-pyrrolidine-2-carbonitrile as well as the uses of this compound have been disclosed. In particular, (2S)-1-{[1,1-Dimethyl-3-(4-pyridin-3-yl-imidazol-1-yl)-propylamino]-acetyl}-pyrrolidine-2-carbonitrile is an inhibitor of DPP-IV and can be used for the treatment and/or prevention of diseases which are associated with DPP-IV, such as diabetes, particularly non-insulin dependent diabetes mellitus, impaired glucose tolerance, bowl disease, colitis ulcerosa, morbus crohn, obesity, and/or metabolic syndrome. The compound can further be used as a diuretic agent or for use as therapeutic active substances for the treatment and/or prophylaxis of hypertension. (2S)-1-{[1,1-Dimethyl-3-(4-pyridin-3-yl-imidazol-1-yl)-propylamino]-acetyl}-pyrrolidine-2-carbonitrile is characterized by formula (I):

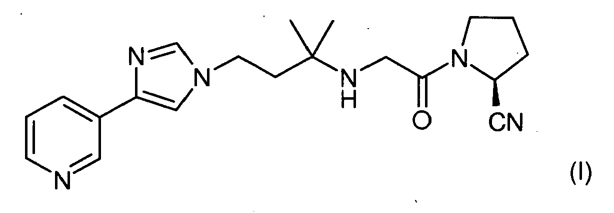

It has now been found that a specific salt of (2S)-1-{[1,1-Dimethyl-3-(4-pyridin-3-yl-imidazol-1-yl)-propylamino]-acetyl}-pyrrolidine-2-carbonitrile, particularly (2S)-1-{[1,1-Dimethyl-3-(4-pyridin-3-yl-imidazol-1-yl)-propylamino]-acetyl}-pyrrolidine-2-carbonitrile fumarate, exhibits unexpected advantages compared to (2S)-1-{[1,1-Dimethyl-3-(4-pyridin-3-yl-imidazol-1-yl)-propylamino]-acetyl}-pyrrolidine-2-carbonitrile. Furthermore, 4 different crystalline polymorphs of (2S)-1-{[1,1-Dimethyl-3-(4-pyridin-3-yl-imidazol-1-yl)-propylamino]-acetyl}-pyrrolidine-2-carbonitrile fumarate have been found, which also exhibit unexpected advantages.

Polymorphism is defined as the ability of a substance to crystallize in more than one crystal lattice arrangement. Polymorphism can influence many aspects of solid state properties of a drug. Different crystal modifications of a substance may differ considerably from one another in many respects such as their solubility, dissolution rate and finally bioavailability. An exhaustive treatment of polymorphism in pharmaceutical and molecular crystals is given e.g. by Byrn (Byrn, S.R., Pfeiffer, R.R., Stowell, J.G., "Solid-State Chemistry of Drugs", SSCI Inc., West Lafayette, Indiana, 1999), Brittain, H.G., "Polymorphism in Pharmaceutical Solids", Marcel Dekker, Inc., New York, Basel, 1999) or Bernstein (Bernstein, J., "Polymorphism in Molecular Crystals", Oxford University Press, 2002).

Surprisingly, it has been found that (2S)-1-{[1,1-Dimethyl-3-(4-pyridin-3-yl-imidazol-1-yl)-propylamino]-acetyl}-pyrrolidine-2-carbonitrile fumarate exhibits various unexpected advantages, e.g. in context with chemical stability, mechanical properties, technical feasibility, processability, solubility, dissolution, bioavailability, toxicology or pharmacokinetic properties.

Surprisingly, it has further been found that (2S)-1-{[1,1-Dimethyl-3-(4-pyridin-3-yl-imidazol-1-yl)-propylamino]-acetyl}-pyrrolidine-2-carbonitrile fumarate can exist in 4 different polymorphic forms, designated as polymorph A, polymorph B, polymorph C and polymorph D, which exhibit various unexpected advantages, e.g. in context with chemical stability, mechanical properties, technical feasibility, processability, solubility, dissolution, bioavailability or pharmacokinetic properties.

Thus, the present invention provides the novel compound (2S)-1-{[1,1-Dimethyl-3-(4-pyridin-3-yl-imidazol-1-yl)-propylamino]-acetyl}-pyrrolidine-2-carbonitrile fumarate and 4 novel crystalline polymorphs of (2S)-1-{[1,1-Dimethyl-3-(4-pyridin-3-yl-imidazol-1-yl)-propylamino]-acetyl}-pyrrolidine-2-carbonitrile fumarate, which unexpectedly exhibit desirable and improved pharmacological properties when compared to the known compound (2S)-1-{[1,1-Dimethyl-3-(4-pyridin-3-yl-imidazol-1-yl)-propylamino]-acetyl}-pyrrolidine-2-carbonitrile.

Unless otherwise indicated, the following definitions are set forth to illustrate and define the meaning and scope of the various terms used to describe the invention herein.

The term "crystalline polymorph" or "polymorph" refers to a crystal form or modification which can be characterized by analytical methods such as e.g. X-ray powder diffraction or IR-spectroscopy.

The term "polymorph A" relates to a specific crystalline polymorph of (2S)-1-{[1,1-Dimethyl-3-(4-pyridin-3-yl-imidazol-1-yl)-propylamino]-acetyl}-pyrrolidine-2-carbonitrile fumarate as defined below.

The term "polymorph B" relates to a specific crystalline polymorph of (2S)-1-{[1,1-Dimethyl-3-(4-pyridin-3-yl-imidazol-1-yl)-propylamino]-acetyl}-pyrrolidine-2-carbonitrile fumarate as defined below.

The term "polymorph C" relates to a specific crystalline polymorph of (2S)-1-{[1,1-Dimethyl-3-(4-pyridin-3-yl-imidazol-1-yl)-propylamino]-acetyl}-pyrrolidine-2-carbonitrile fumarate as defined below.

The term "polymorph D" relates to a specific crystalline polymorph of (2S)-1-{[1,1-Dimethyl-3-(4-pyridin-3-yl-imidazol-1-yl)-propylamino]-acetyl}-pyrrolidine-2-carbonitrile fumarate as defined below.

The term "IR" means infrared.

Description of Figures:
Figure 1: X-ray diffraction pattern of polymorph A
Figure 2: IR-spectrum of polymorph A
Figure 3: X-ray diffraction pattern of polymorph B
Figure 4: IR-spectrum of polymorph B
Figure 5: X-ray diffraction pattern of polymorph C
Figure 6: IR-spectrum of polymorph C
Figure 7: X-ray diffraction pattern of polymorph D
Figure 8: IR-spectrum of polymorph D

In detail, the present invention relates to the compound (2S)-1-{[1,1-Dimethyl-3-(4-pyridin-3-yl-imidazol-1-yl)-propylamino]-acetyl}-pyrrolidine-2-carbonitrile fumarate.

Furthermore, the present invention relates to a crystalline polymorph of the compound as described above, which is characterized by an X-ray powder diffraction pattern having characteristic peaks expressed in degrees 2-theta at approximately

| **degree 2-theta** |
|---|
| 9.0 |
| 10.8 |
| 14.1 |
| 19.6 |
| 21.6 |

This polymorph is referred to as "polymorph A". The term "approximately" means in this context that there is an uncertainty in the measurements of the degrees 2-theta of ± 0.2 (expressed in degrees 2-theta).

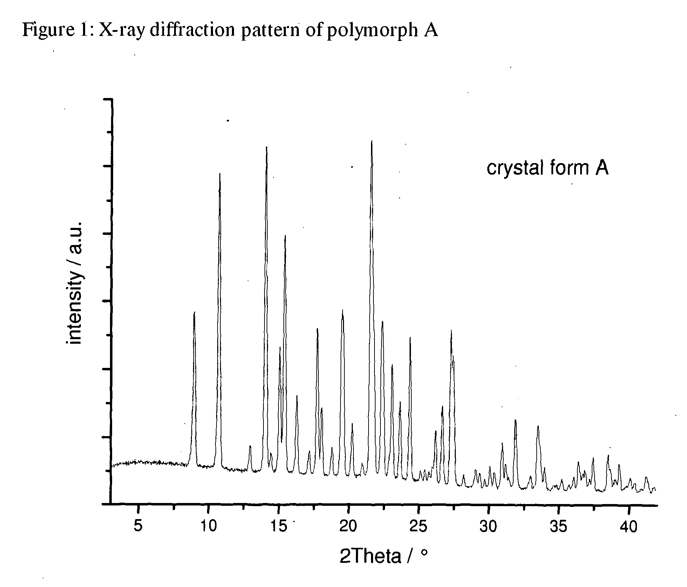

Preferably, the crystalline polymorph A as defined above is characterized by an X-ray powder diffraction pattern having characteristic peaks expressed in degrees 2-theta at approximately 9.0, 10.8, 13.0, 14.1, 14.4, 15.1, 15.4, 16.3, 17.2, 17.8, 18.1, 18.8, 19.6, 20.2, 21.6, 22.4, 23.1, 23.7, 24.4, 26.2, 26.7, 27.3, 27.5, 31.0, 31.9 and 33.5. More preferably, the crystalline polymorph A as defined above is characterized by the X-ray powder diffraction pattern shown in figure 1.

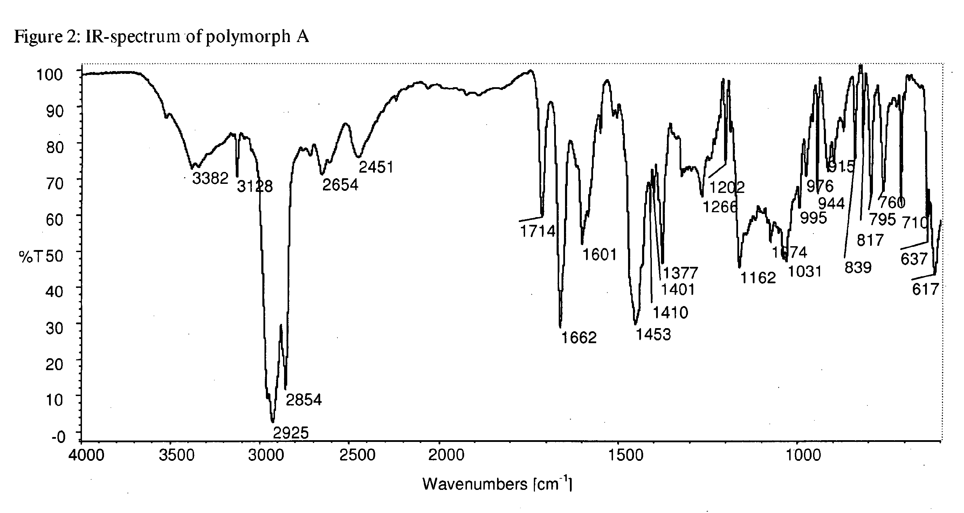

The crystalline polymorph A as described above can also be characterized by its IR-spectrum. The present invention therefore also relates to a crystalline polymorph of the compound as defined above, which is characterized by an IR absorption spectrum having characteristic peaks at approximately 3382 cm⁻¹, 3128 cm⁻¹, 2654 cm⁻¹, 2451 cm⁻¹, 1714 cm⁻¹, 1662 cm⁻¹, 1601 cm⁻¹, 1266 cm⁻¹, 1202 cm⁻¹, 1162 cm⁻¹, 1074 cm⁻¹, 1031 cm⁻¹, 995 cm⁻¹, 976 cm⁻¹, 944 cm⁻¹, 915 cm⁻¹, 839 cm⁻¹, 817 cm⁻¹, 760 cm⁻¹, 710 cm⁻¹ and 637 cm⁻¹. The term "approximately" means in this context that the cm⁻¹ values can vary, e.g. by up to ± 1 cm⁻¹. Preferably, the crystalline polymorph A as described above, is characterized by the IR absorption spectrum shown in figure 2.

Another embodiment of the present invention is related to a crystalline polymorph of the compound as defined above, which is characterized by an X-ray powder diffraction pattern having characteristic peaks expressed in degrees 2-theta at approximately

| **degree 2-theta** |
|---|
| 10.3 |
| 16.0 |
| 17.3 |
| 19.0 |
| 21.3 |

This polymorph is referred to as "polymorph B". The term "approximately" means in this context that there is an uncertainty in the measurements of the degrees 2-theta of ± 0.2 (expressed in degrees 2-theta).

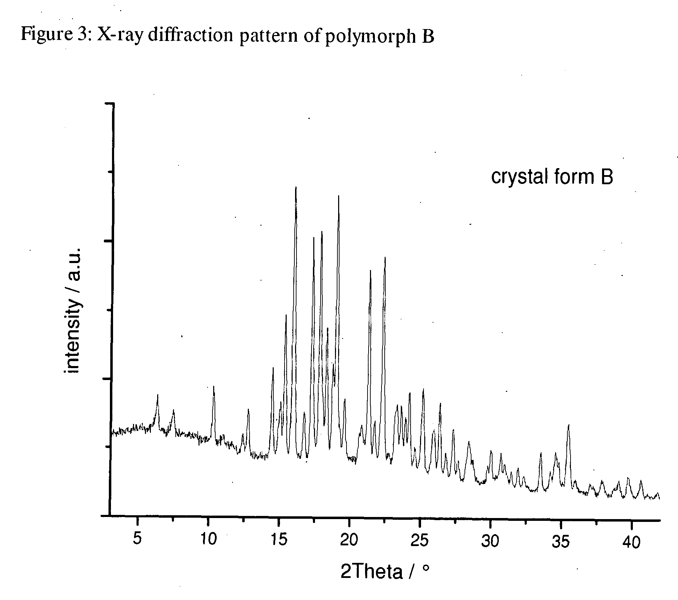

Preferably, the crystalline polymorph B as defined above is characterized by an X-ray powder diffraction pattern having characteristic peaks expressed in degrees 2-theta at approximately 6.3, 7.5, 10.3, 12.8, 14.5, 15.1, 15.4, 16.0, 16.7, 17.3, 17.9, 18.3, 18.8, 19.0, 19.6, 21.3, 21.8, 22.3, 23.3, 23.6, 23.9, 24.2, 25.2, 26.4 and 35.5. More preferably, the crystalline polymorph B as defined above is characterized by the X-ray powder diffraction pattern shown in figure 3.

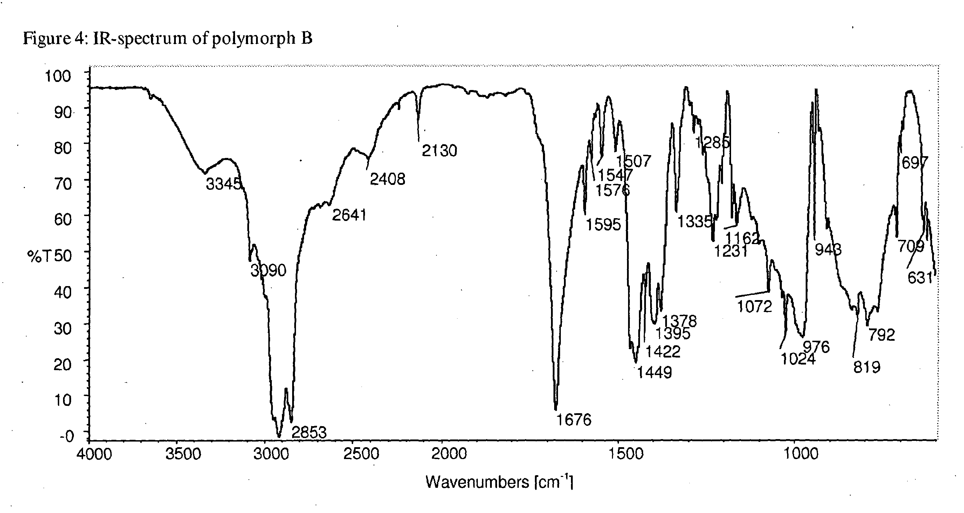

The crystalline polymorph B as described above can also be characterized by its IR-spectrum. The present invention therefore also relates to a crystalline polymorph of the compound as defined above, which is characterized by an IR absorption spectrum having characteristic peaks at approximately 3345 cm⁻¹, 3090 cm⁻¹, 2641 cm⁻¹, 2408 cm⁻¹, 2130 cm⁻¹, 1676 cm⁻¹, 1595 cm⁻¹, 1335 cm⁻¹, 1231 cm⁻¹, 1162 cm⁻¹, 1072 cm⁻¹, 1024 cm⁻¹, 976 cm⁻¹, 943 cm⁻¹, 819 cm⁻¹, 792 cm⁻¹, 709 cm⁻¹ and 631 cm⁻¹. The term "approximately" means in this context that the cm⁻¹ values can vary, e.g. by up to ± 1 cm⁻¹. Preferably, the crystalline polymorph B as described above, is characterized by the IR absorption spectrum shown in figure 4.

Furthermore, the present invention relates to a crystalline polymorph of the compound as described above, which is characterized by an X-ray powder diffraction pattern having characteristic peaks expressed in degrees 2-theta at approximately

| **degree 2-theta** |
|---|
| 6.4 |
| 11.0 |
| 11.5 |
| 14.0 |
| 19.6 |

This polymorph is referred to as "polymorph C". The term "approximately" means in this context that there is an uncertainty in the measurements of the degrees 2-theta of ± 0.2 (expressed in degrees 2-theta).

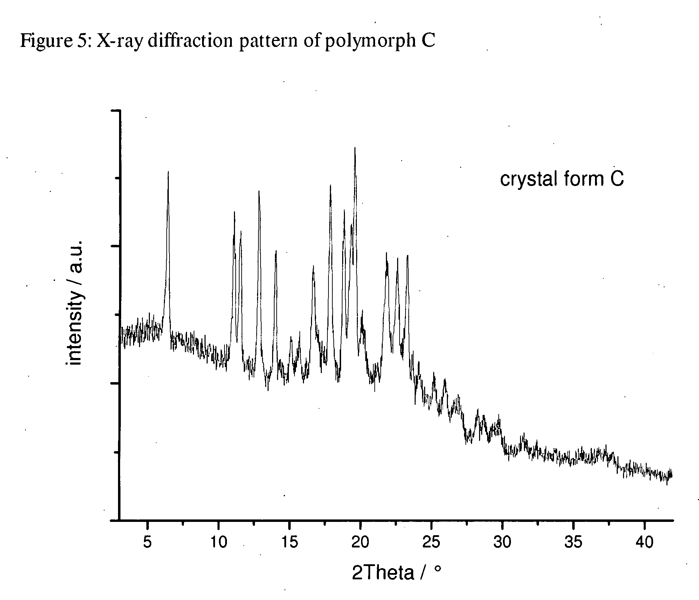

Preferably, the crystalline polymorph C as defined above is characterized by an X-ray powder diffraction pattern having characteristic peaks expressed in degrees 2-theta at approximately 6.4, 11.0, 11.5, 12.8, 14.0, 16.7, 17.8, 18.8, 19.3, 19.6, 20.1, 21.8, 22.6 and 23.3. More preferably, the crystalline polymorph C as defined above is characterized by the X-ray powder diffraction pattern shown in figure 5.

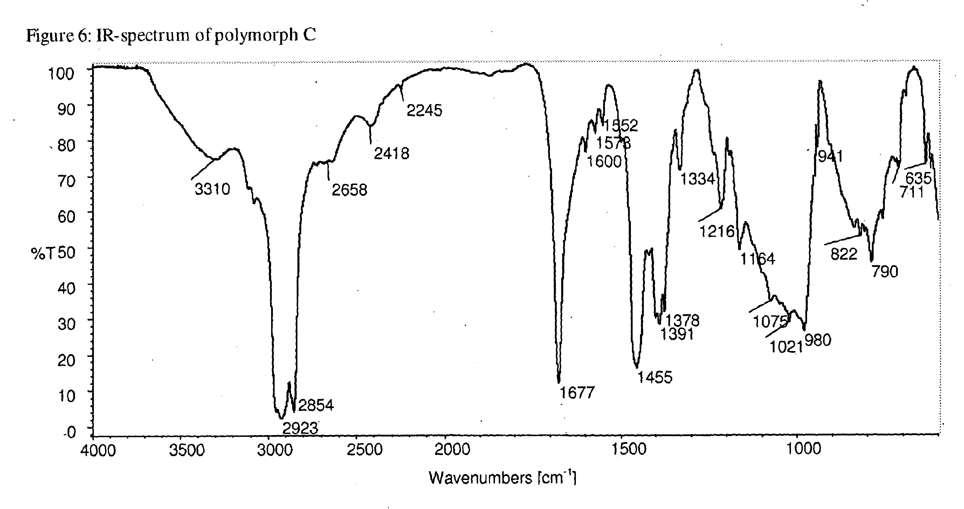

The crystalline polymorph C as described above can also be characterized by its IR-spectrum. The present invention therefore also relates to a crystalline polymorph of the compound as defined above, which is characterized by an IR absorption spectrum having characteristic peaks at approximately 3310 cm⁻¹, 2658 cm⁻¹, 2418 cm⁻¹, 2245 cm⁻¹, 1677 cm⁻¹, 1600 cm⁻¹, 1573 cm⁻¹, 1334 cm⁻¹, 1216 cm⁻¹, 1164 cm⁻¹, 1075 cm⁻¹1021 cm⁻¹, 960 cm⁻¹, 941 cm⁻¹, 822 cm⁻¹, 790 cm⁻¹, 711 cm⁻¹ and 635 cm⁻¹. The term "approximately" means in this context that the cm⁻¹ values can vary, e.g. by up to ± 1 cm⁻¹. Preferably, the crystalline polymorph C as described above, is characterized by the IR absorption spectrum shown in figure 6.

Furthermore, the present invention relates to a crystalline polymorph of the compound as described above, which is characterized by an X-ray powder diffraction pattern having characteristic peaks expressed in degrees 2-theta at approximately

| **degree 2-theta** |
|---|
| 7.1 |
| 10.7 |
| 14.4 |
| 15.6 |
| 17.6 |

This polymorph is referred to as "polymorph D". The term "approximately" means in this context that there is an uncertainty in the measurements of the degrees 2-theta of ± 0.2 (expressed in degrees 2-theta).

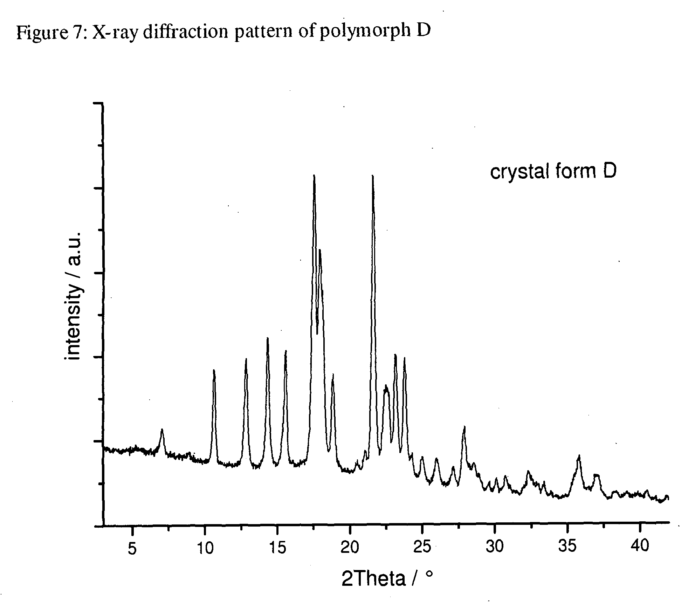

Preferably, the crystalline polymorph D as defined above is characterized by an X-ray powder diffraction pattern having characteristic peaks expressed in degrees 2-theta at approximately 7.1, 10.7, 12.9, 14.4, 15.6, 17.6, 18.0, 18.9, 21.6, 22.6, 23.2, 23.8 and 27.9. More preferably, the crystalline polymorph D as defined above is characterized by the X-ray powder diffraction pattern shown in figure 7.

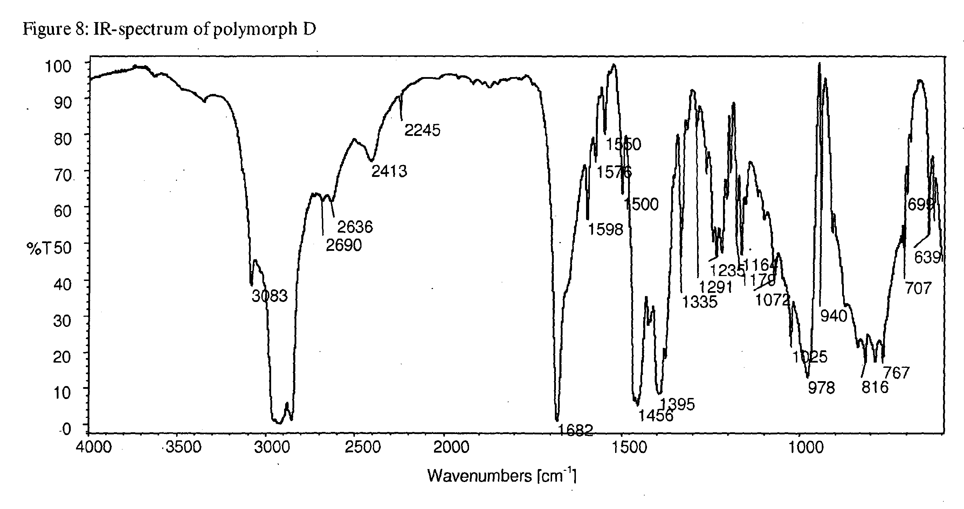

The crystalline polymorph D as described above can also be characterized by its IR-spectrum. The present invention therefore also relates to a crystalline polymorph of the compound as defined above, which is characterized by an IR absorption spectrum having characteristic peaks at approximately 3083 cm⁻¹, 2690 cm⁻¹, 2636 cm⁻¹, 2413 cm⁻¹, 22.45 cm⁻¹, 1682 cm⁻¹, 1598 cm⁻¹, 1576 cm⁻¹, 1550 cm⁻¹, 1500 cm⁻¹, 1335 cm⁻¹, 1291 cm⁻¹, 1235 cm⁻¹, 1179 cm⁻¹, 1164 cm⁻¹, 1072 cm⁻¹, 1025 cm⁻¹, 978 cm⁻¹, 940 cm⁻¹, 816 cm⁻¹, 767 cm⁻¹, 707 cm⁻¹ and 639 cm⁻¹. The term "approximately" means in this context that the cm⁻¹ values can vary, e.g. by up to ± 1 cm⁻¹. Preferably, the crystalline polymorph D as described above, is characterized by the IR absorption spectrum shown in figure 8.

The degrees 2-theta values mentioned above refer to measurements with Cu Kα1 radiation at 20 - 25 °C.

Moreover, the invention relates especially to the compound (2S)-1-{[1,1-Dimethyl-3-(4-pyridin-3-yl-imidazol-1-yl)-propylamino]-acetyl}-pyrrolidine-2-carbonitrile fumarate, wherein at least 70% are a crystalline polymorph as defined above, particularly wherein at least 90% are a crystalline polymorph as defined above, more particularly wherein at least 95% are a crystalline polymorph as defined above and even more particularly wherein at least 99% are a crystalline polymorph as defined above.

In a preferred embodiment, the present invention relates to a process for the preparation of a compound as defined above, which process comprises reacting (2S)-1-{[1,1-Dimethyl-3-(4-pyridin-3-yl-imidazol-1-yl)-propylamino]-acetyl}-pyrrolidine-2-carbonitrile with fumaric acid. This conversion is preferably carried out with fumaric acid in a suitable solvent such as e.g. ethanol, methanol, propanol, THF, isopropanol, isopropanol/water, ethanol/THF, propanol/water. The fumarate salt can then be obtained by subsequent crystallization.

Another preferred embodiment of the present invention relates to a process as defined above, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from 1-propanol in the presence of water. Preferably, 6 % to 10 % (w/w), more preferably 7 % to 9 % (w/w), more preferably 8 % (w/w), of (2S)-1-{[1,1-Dimethyl-3-(4-pyridin-3-yl-imidazol-1-yl)-propylamino]-acetyl}-pyrrolidine-2-carbonitrile fumarate are dissolved in 1-propanol in the presence of 5 % to 9 %(w/w), more preferably 6 % to 8 % (w/w) , more preferably 7 % (w/w) water, preferably at a temperature of 65 °C to 75 °C, more preferably 70 °C, and crystallized by cooling. This leads to polymorph A. Preferably, previously obtained polymorph A is added, in order to facilitate the formation of the desired polymorph. The present invention also relates to a process for the preparation of polymorph A, which process comprises crystallizing (2S)-1-{[1,1-Dimethyl-3-(4-pyridin-3-yl-imidazol-1-yl)-propylamino]-acetyl}-pyrrolidine-2-carbonitrile fumarate from 1-propanol in the presence of water.

Another preferred embodiment of the present invention relates to a process as defined above, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from 1-propanol in the absence of water. Preferably, 6 % to 10 % (w/w), more preferably 7 % to 9 % (w/w), more preferably 8 % (w/w), of (2S)-1-{[1,1-Dimethyl-3-(4-pyridin-3-yl-imidazol- 1-yl)-propylamino]-acetyl}-pyrrolidine-2-carbonitrile fumarate are dissolved in 1-propanol in the absence of water, preferably at a temperature of 55 °C to 70 °C, more preferably 60 °C to 65 °C, and crystallized by cooling. This leads to polymorph B. The present invention also relates to a process for the preparation of polymorph B, which process comprises crystallizing (2S)-1-{[1,1-Dimethyl-3-(4-pyridin-3-yl-imidazol-1-yl)-propylamino]-acetyl}-pyrrolidine-2-carbonitrile fumarate from 1-propanol in the absence of water.

Another preferred embodiment of the present invention relates to a process as defined above, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from isopropanol. Preferably, the fumarate salt is formed in isopropanol with subsequent crystallisation of the desired polymorph. Preferably, 9 % to 13 % (w/w), more preferably about 10 % to 12 % (w/w), more preferably 11 % (w/w), of (2S)-1-{[1,1-Dimethyl-3-(4-pyridin-3-yl-imidazol-1-yl)-propylamino]-acetyl}-pyrrolidine-2-carbonitrile are dissolved in isopropanol, preferably at a temperature of 30 °C to 40 °C, more preferably 35 °C, and combined with the same volume of an equimolar solution of fumaric acid in isopropanol, preferably at a temperature of 30 °C to 40 °C, more preferably 35 °C. The crystal slurry is diluted with isopropanol and cooled to ambient temperature. This leads to polymorph C.

Another preferred embodiment of the present invention relates to a process as defined above, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from THF. Preferably, 9 % to 13 % (w/w), more preferably 10 % to 12 % (w/w), more preferably 11 % (w/w), of (2S)-1-{[1,1-Dimethyl-3-(4-pyridin-3-yl-imidazol-1-yl)-propylamino]-acetyl}-pyrrolidine-2-carbonitrile are dissolved in THF, preferably at a temperature of 55 °C to 65 °C, more preferably 60 °C, and combined with the same volume of an equimolar solution of fumaric acid in THF, preferably at a temperature of 55 °C to 65 °C, more preferably 60 °C, crystallized by cooling and diluted with THF. This leads to polymorph D.

Furthermore, the invention relates to a compound or crystalline polymorph as defined above, when manufactured by a process as described above.

As described above, the compounds and/or polymorphs of the present invention can be used as medicaments for the treatment and/or prophylaxis of diseases which are associated with DPP-IV such as diabetes, particularly non-insulin dependent diabetes mellitus, impaired glucose tolerance, bowl disease, colitis ulcerosa, morbus crohn, obesity, and/or metabolic syndrome, preferably non-insulin dependent diabetes mellitus and/or impaired glucose tolerance. Furthermore, the compounds and/or polymorphs of the present invention can be used as diuretic agents or for the treatment and/or prophylaxis of hypertension.

The invention therefore also relates to pharmaceutical compositions comprising a compound or crystalline polymorph as defined above and a pharmaceutically acceptable carrier and/or adjuvant.

Further, the invention relates to a compound or crystalline polymorph as defined above for use as therapeutic active substance, particularly as therapeutic active substance for the treatment and/or prophylaxis of diseases which are associated with DPP-IV such as diabetes, particularly non-insulin dependent diabetes mellitus, impaired glucose tolerance, bowl disease, colitis ulcerosa, morbus crohn, obesity, and/or metabolic syndrome, preferably for use as therapeutic active substances for the treatment and/or prophylaxis of non-insulin dependent diabetes mellitus and/or impaired glucose tolerance. The invention relates furthermore to a compound or crystalline polymorph as defined above for use as diuretic agents or for use as therapeutic active substance for the treatment and/or prophylaxis of hypertension.

The invention also relates to a method for the treatment and/or prophylaxis of diseases which are associated with DPP-IV such as diabetes, particularly non-insulin dependent diabetes mellitus, impaired glucose tolerance, bowl disease, colitis ulcerosa, morbus crohn, obesity, and/or metabolic syndrome, preferably for the treatment and/or prophylaxis of non-insulin dependent diabetes mellitus and/or impaired glucose tolerance, which method comprises administering a compound or crystalline polymorph as defined above to a human being or animal. The invention relates furthermore to a method for the treatment and/or prophylaxis as defined above, wherein the disease is hypertension or wherein a diuretic agent has a beneficial effect.

The invention further relates to the use of a compound or crystalline polymorph as defined above for the treatment and/or prophylaxis of diseases which are associated with DPP-IV such as diabetes, particularly non-insulin dependent diabetes mellitus, impaired glucose tolerance, bowl disease, colitis ulcerosa, morbus crohn, obesity, and/or metabolic syndrome, preferably for the treatment and/or prophylaxis of non-insulin dependent diabetes mellitus and/or impaired glucose tolerance. The invention relates furthermore to the use as defined above, wherein the disease is hypertension or to the use as diuretic agent.

In addition, the invention relates to the use of a compound or crystalline polymorph as defined above for the preparation of medicaments for the treatment and/or prophylaxis of diseases which are associated with DPP-IV such as diabetes, particularly non-insulin dependent diabetes mellitus, impaired glucose tolerance, bowl disease, colitis ulcerosa, morbus crohn, obesity, and/or metabolic syndrome, preferably for the treatment and/or prophylaxis of non-insulin dependent diabetes mellitus and/or impaired glucose tolerance. Such medicaments comprise a compound as defined above. The invention relates furthermore to the use as defined above, wherein the disease is hypertension or the use for the preparation of diuretic agents.

In context with the methods and uses defined above, the following diseases relate to a preferred embodiment: diabetes, particularly non-insulin dependent diabetes mellitus, impaired glucose tolerance, obesity, and/or metabolic syndrome, preferably non-insulin dependent diabetes mellitus and/or impaired glucose tolerance.

In the compositions, uses and methods as described above, the compound (2S)-1-{[1,1-Dimethyl-3-(4-pyridin-3-yl-imidazol-1-yl)-propylamino]-acetyl}-pyrrolidine-2-carbonitrile fumarate, the polymorphs as described above, or the compound (2S)-1-{[1,1-Dimethyl-3-(4-pyridin-3-yl-imidazol-1-yl)-propylamino]-acetyl}-pyrrolidine-2-carbonitrile fumarate wherein at least 70% are a crystalline polymorph as defined above, particularly wherein at least 90% are a crystalline polymorph as defined above, more particularly wherein at least 95% are a crystalline polymorph as defined above and even more particularly wherein at least 99% are a crystalline polymorph as defined above, can be used.

The following tests were carried out in order to determine the activity of the compounds and crystalline polymorphs as described above.

Activity of DPP-IV inhibitors are tested with natural human DPP-IV derived from a human plasma pool or with recombinant human DPP-IV. Human citrate plasma from different donors is pooled, filtered through a 0.2 micron membrane under sterile conditions and aliquots of 1 mL are shock frozen and stored at -120 °C until used. In the colorimetric DPP-IV assay 5 to 10 µL human plasma and in the fluorometric assay 1.0 µL of human plasma in a total assay volume of 100 µL is used as an enzyme source. The cDNA of the human DPP-IV sequence of amino acid 31 - to 766, restricted for the N-terminus and the transmembrane domain, is cloned into pichia pastoris. Human DPP-IV is expressed and purified from the culture medium using conventional column chromatography including size exclusion and anion and cation chromatography. The purity of the final enzyme preparation of Coomassie blue SDS-PAGE is >95%. In the colorimetric DPP-IV assay 20 ng rec.-h DPP-IV and in the fluorometric assay 2 ng rec-h DPP-IV in a total assay volume of 100 µL is used as an enzyme source.

In the fluorogenic assay Ala-Pro-7-amido-4-trifluoromethylcoumarin (Calbiochem No 125510) is used as a substrate. A 20 mM stock solution in 10 % DMF/H₂O is stored at -20 °C until use. In IC50 determinations a final substrate concentration of 50 µM is used. In assays to determine kinetic parameters as Km, Vmax, Ki, the substrate concentration is varied between 10 µM and 500 µM.

In the colorimetric assay H-Ala-Pro-pNA.HCl (Bachem L-1115) is used as a substrate. A 10 mM stock solution in 10% MeOH/H₂O is stored at -20 °C until use. In IC50 determinations a final substrate concentration of 200 µM is used. In assays to determine kinetic parameters as Km, Vmax, Ki, the substrate concentration is varied between 100 µM and 2000 µM. Fluorescence is detected in a Perkin Elmer Luminescence Spectrometer LS 50B at an excitation wavelength of 400 nm and an emission wavelength of 505 nm continuously every 15 seconds for 10 to 30 minutes. Initial rate constants are calculated by best fit linear regression. The absorption of pNA liberated from the colorimetric substrate is detected in a Packard SpectraCount at 405 nM continuosly every 2 minutes for 30 to 120 minutes. Initial rate constants are calculated by best fit linear regression.

DPP-IV activity assays are performed in 96 well plates at 37 °C in a total assay volume of 100 µl. The assay buffer consists of 50 mM Tris/HCl pH 7.8 containing 0.1 mg/mL BSA and 100 mM NaCl. Test compounds are dissolved in 100 % DMSO, diluted to the desired concentration in 10% DMSO/H₂O. The final DMSO concentration in the assay is 1% (v/v). At this concentration enzyme inactivation by DMSO is <5%. Compounds are with (10 minutes at 37 °C) and without preincubation with the enzyme. Enzyme reactions are started with substrate application followed by immediate mixing.

IC50 determinations of test compounds are calculated by non-linear best fit regression of the DPP-IV inhibition of at least 5 different compound concentrations. Kinetic parameters of the enzyme reaction are calculated from at least 5 different substrate concentrations and at least 5 different test compound concentrations.

The compounds and crystalline polymorphs of the present invention exhibit IC50 values in the range of 10 nM to 500 nM, more preferably of 50 - 100 nM.

| Compound | IC₅₀ [nM] | Ki [nM] |
|---|---|---|
| (2S)-1-{[1,1-Dimethyl-3-(4-pyridin-3-yl-imidazol-1-yl)-propylamino]-acetyl}-pyrrolidine-2-carbonitrile fumarate | 21.2 | 22.7 |

The compounds and polymorphs of the present invention can be used as medicament, e.g. in the form of pharmaceutical preparations for enteral, parenteral or topical administration. It can be administered, for example, perorally, e.g. in the form of tablets, coated tablets, dragées, hard and soft gelatine capsules, solutions, emulsions or suspensions, rectally, e.g. in the form of suppositories, parenterally, e.g. in the form of injection solutions or suspensions or infusion solutions, or topically, e.g. in the form of ointments, creams or oils.

The production of the pharmaceutical preparations can be effected in a manner which will be familiar to any person skilled in the art by bringing the described polymorph, optionally in combination with other therapeutically valuable substances, into a galenical administration form together with suitable, non-toxic, inert, therapeutically compatible solid or liquid carrier materials and, if desired, usual pharmaceutical adjuvants.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers might, however, be required in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds and polymorphs of the present invention can vary within wide limits depending on the disease to be controlled, the age and the individual condition of the patient and the mode of administration, and will, of course, be fitted to the individual requirements in each particular case. For adult patients a daily dosage of about 50 to 2000 mg, especially about 200 to 1000 mg, comes into consideration. Depending on severity of the disease and the precise pharmacokinetic profile the compounds and polymorphs of the present invention could be administered with one or several daily dosage units, e.g. in 1 to 3 dosage units.

The pharmaceutical preparations conveniently contain about 50 to 1000 mg, preferably 200 to 500 mg, of a compound and/or polymorph of the present invention.

The following examples serve to illustrate the present invention in more detail. They are, however, not intended to limit its scope in any manner. The compound (2S)-1-{[1,1-Dimethyl-3-(4-pyridin-3-yl-imidazol-1-yl)-propylamino]-acetyl}-pyrrolidine-2-carbonitrile can be obtained by the methods disclosed in WO 03/037327 or in analogy thereto. The compound (2S)-1-{[1,1-Dimethyl-3-(4-pyridin-3-yl-imidazol-1-yl)-propylamino]-acetyl}-pyrrolidine-2-carbonitrile fumarate can be obtained by the methods given above or in the examples or by methods generally known to the person skilled in the art. The polymorphs according to the present invention can be manufactured by the methods given above, by the methods given in the examples or by analogous methods. Starting materials are either commercially available or can be prepared by methods analogous to the methods given above or in the examples or by methods known in the art.

### Examples

### X-ray Powder Diffraction

The X-ray powder diffraction patterns were recorded with a STOE Stadi P X-ray diffractometer in transmission mode (Cu Kα1 radiation, Ge-monochromator, position sensitive detector (PSD), angular range 3° to 42° 2-theta, steps of 0.5° 2Theta, measuring time 40 seconds per step). The samples were prepared and analyzed without further processing (e.g. grinding or sieving) of the substance at ambient temperature (20 - 25 °C).

### IR-Spectroscopy

The IR-spectra of the samples were recorded as film of a Nujol suspension consisting of approx. 15 mg of sample and approx. 15 mg of Nujol between two sodium chloride plates, with an FT-IR spectrometer in transmittance. The Spectrometer is a Nicolet 20SXB or equivalent (resolution 2 cm⁻¹, 32 or 64 coadded scans, MCT detector).

### Example 1

200 mg of (2S)-1-{[1,1-Dimethyl-3-(4-pyridin-3-yl-imidazol-1-yl)-propylamino]-acetyl}-pyrrolidine-2-carbonitrile (chromatographed free base, 546 µmol) were dissolved in 2.0 mL of 1-propanol. At ambient temperature 65 mg (ca. 1.1 equivalents) of solid fumaric acid were added. The thickening slurry was stirred for 30 minutes, before 100 µL of water were added. The suspension was heated to 65 °C until a clear solution was obtained. The solution was linearly cooled to 20 °C within 3 h and then aged at this temperature over night. Prior to filtration the suspension was diluted with 2.0 mL of propanol. The filter cake was rinsed with 3.0 mL of 1-propanol. After drying (25 °C, 5 mbar, 15 h) 220 mg (83.5%) off white (2S)-1-{[1,1-Dimethyl-3-(4-pyridin-3-yl-imidazol-1-yl)-propylamino]-acetyl}-pyrrolidine-2-carbonitrile fumarate were obtained as fine needles, in the form of polymorph A.

### Example 2

1.0 g of crude (2S)-1-{[1,1-Dimethyl-3-(4-pyridin-3-yl-imidazol-1-yl)-propylamino]-acetyl}-pyrrolidine-2-carbonitrile fumarate were dissolved at 70 °C in a mixture of 15 mL of 1-propanol and 600 uL of water. After polishing filtration the solution was linearly cooled from 70 °C to 20 °C without agitation within 8 h. The crystals were then mobilized with a spatula and the suspension was stirred at 20 °C for additional 8 h. The product was collected by filtration, rinsed twice with 5 mL of propanol and then dried in vacuum (5 mbar) at ambient temperature over night, yielding 805 mg (81%) (2S)-1-{[1,1-Dimethyl-3-(4-pyridin-3-yl-imidazol-1-yl)-propylamino]-acetyl}-pyrrolidine-2-carbonitrile fumarate as crystalline polymorph A as off-white needles. Mp. 180 °C

### Example 3

1.0 g of crude (2S)-1-{[1,1-Dimethyl-3-(4-pyridin-3-yl-imidazol-1-yl)-propylamino]-acetyl}-pyrrolidine-2-carbonitrile fumarate (not in form A) were dissolved in 15 mLof 1-propanol at 65 °C. After polishing filtration the solution was linearly cooled from 65 °C to 15 °C without agitation within 8 h. The crystals were mobilized with a spatula and the suspension was stirred at 15 °C for additional 5 h. The product was collected by filtration, rinsed twice with 5 mL of cold 1-propanol and then dried in vacuum (5 mbar) at ambient temperature over night, yielding 830 mg (83%) (2S)-1-{[1,1-Dimethyl-3-(4-pyridin-3-yl-imidazol-1-yl)-propylamino]-acetyl}-pyrrolidine-2-carbonitrile fumarate as crystalline polymorph B as slightly beige powder. Mp. 162 °C

### Example 4

200 mg of (2S)-1-{[1,1-Dimethyl-3-(4-pyridin-3-yl-imidazol-1-yl)-propylamino]-acetyl}-pyrrolidine-2-carbonitrile (chromatographed free base, 546 µmol) were dissolved in 2 mL of isopropanol at ambient temperature. Then a warm (30-40 °C) solution of 65 mg of fumaric acid (560 µmol, 1.03 equivalents) in 2 mL of isopropanol was added rapidly. The resulting thick suspension was diluted with 5 mL of isopropanol and stirred over night at ambient temperature. The product was collected by filtration, rinsed twice with 5 mL of isopropanol and then dried in vacuum (5 mbar) at ambient temperature over night, yielding 230 mg (87%) (2S)-1-{[1,1-Dimethyl-3-(4-pyridin-3-yl-imidazol-1-yl)-propylamino]-acetyl}-pyrrolidine-2-carbonitrile fumarate as crystalline polymorph C as beige powder.

### Example 5

200 mg of (2S)-1-{[1,1-Dimethyl-3-(4-pyridin-3-yl-imidazol-1-yl)-propylamino]-acetyl}-pyrrolidine-2-carbonitrile (chromatographed free base, 546 µmol) were dissolved in 2 mL of THF. At 60 °C a solution of 65 mg of fumaric acid (560 µmol, 1.03 equivalents) in 2 mL of of THF was added slowly. The resulting suspension was then passively cooled to ambient temperature and then stirred over night. The resulting thick suspension was diluted with 5 mL of THF before the product was collected by filtration. The crop was rinsed twice with 5 mL of THF and then dried in vacuum (5 mbar) at ambient temperature over night, yielding 197 mg (76%) (2S)-1-{[1,1-Dimethyl-3-(4-pyridin-3-yl-imidazol-1-yl)-propylamino]-acetyl}-pyrrolidine-2-carbonitrile fumarate as crystalline polymorph D as beige powder. Mp. 156 °C.

### Example A

Film coated tablets containing the following ingredients can be manufactured in a conventional manner:

| Ingredients | Per tablet | |
|---|---|---|
| Kernel: | | |
| Active ingredient | 50.0 mg | 200.0 mg |
| Lactose hydrous | 58.5 mg | 125.5 mg |
| Povidone K30 | 10 mg | 20.0 mg |
| Magnesium stearate | 1.5 mg | 4.5 mg |
| (Kernel Weight) | 120.0 mg | 350.0 mg |
| Film Coat: | | |
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Titan dioxide | 0.8 mg | 1.6 mg |

The active ingredient is sieved and mixed with lactose and the mixture is granulated with a solution of polyvinylpyrrolidone in water or ethanol by fluid bed granulation. The granulate is mixed with sodium starch glycolate and magnesium stearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Active ingredient | 25.0 mg |
| Lactose | 170.0 mg |
| Talc | 5.0 mg |

The components are sieved and mixed and filled into capsules of size 2.

## Claims

1. The compound (2S)-1-{[1,1-Dimethyl-3-(4-pyridin-3-yl-imidazol-1-yl)-propylamino] -acetyl }-pyrrolidine-2-carbonitrile fumarate.

2. A crystalline polymorph of the compound according to claim 1, which is **characterized by** an X-ray powder diffraction pattern having characteristic peaks expressed in degrees 2-theta at approximately
| **degree 2-theta** |
|---|
| 9.0 |
| 10.8 |
| 14.1 |
| 19.6 |
| 21.6 |

3. The crystalline polymorph according to claim 2, **characterized by** the X-ray powder diffraction pattern shown in figure 1.

4. A crystalline polymorph of the compound according to claim 1, which is **characterized by** an IR absorption spectrum having characteristic peaks at approximately 3382 cm⁻¹, 3128 cm⁻¹, 2654 cm⁻¹, 2451 cm⁻¹, 1714 cm⁻¹, 1662 cm⁻¹, 1601 cm⁻¹, 1266 cm⁻¹, 1202 cm⁻¹, 1162 cm⁻¹, 1074 cm⁻¹, 1031 cm⁻¹, 995 cm⁻¹, 976 cm⁻¹, 944 cm⁻¹, 915 cm⁻¹, 839 cm⁻¹, 817 cm⁻¹, 760 cm⁻¹, 710 cm⁻¹ and 637 cm⁻¹.

5. The crystalline polymorph of claim 4, **characterized by** the IR absorption spectrum shown in figure 2.

6. A crystalline polymorph of the compound according to claim 1, which is **characterized by** an X-ray powder diffraction pattern having characteristic peaks expressed in degrees 2-theta at approximately
| **degree 2-theta** |
|---|
| 10.3 |
| 16.0 |
| 17.3 |
| 19.0 |
| 21.3 |

7. The crystalline polymorph according to claim 6, **characterized by** the X-ray powder diffraction pattern shown in figure 3.

8. A crystalline polymorph of the compound according to claim 1, which is **characterized by** an IR absorption spectrum having characteristic peaks at approximately 3345 cm⁻¹, 3090 cm⁻¹, 2641 cm⁻¹, 2408 cm⁻¹, 2130 cm⁻¹, 1676 cm⁻¹, 1595 cm⁻¹, 1335 cm⁻¹, 1231 cm⁻¹, 1162 cm⁻¹, 1072 cm⁻¹, 1024 cm⁻¹, 976 cm⁻¹, 943 cm⁻¹, 819 cm⁻¹, 792 cm⁻¹, 709 cm⁻¹ and 631 cm⁻¹.

9. A crystalline polymorph of the compound according to claim 8, which is **characterized by** the IR absorption spectrum shown in figure 4.

10. A crystalline polymorph of the compound according to claim 1, which is **characterized by** an X-ray powder diffraction pattern having characteristic peaks expressed in degrees 2-theta at approximately
| **degree 2-theta** |
|---|
| 6.4 |
| 11.0 |
| 11.5 |
| 14.0 |
| 19.6 |

11. The crystalline polymorph according to claim 10, **characterized by** the X-ray powder diffraction pattern shown in figure 5.

12. A crystalline polymorph of the compound according to claim 1, which is **characterized by** an IR absorption spectrum having characteristic peaks at approximately 3310 cm⁻¹, 2658 cm⁻¹, 2418 cm⁻¹, 2245 cm⁻¹, 1677 cm⁻¹, 1600 cm⁻¹, 1573 cm⁻¹, 1334 cm⁻¹, 1216 cm⁻¹, 1164 cm⁻¹, 1075 cm⁻¹, 1021 cm⁻¹, 960 cm⁻¹, 941 cm⁻¹, 822 cm⁻¹, 790 cm⁻¹, 711 cm⁻¹ and 635 cm⁻¹.

13. The crystalline polymorph of claim 12, **characterized by** the IR absorption spectrum shown in figure 6.

14. A crystalline polymorph of the compound according to claim 1, which is **characterized by** an X-ray powder diffraction pattern having characteristic peaks expressed in degrees 2-theta at approximately
| **degree 2-theta** |
|---|
| 7.1 |
| 10.7 |
| 14.4 |
| 15.6 |
| 17.6 |

15. The crystalline polymorph according to claim 14, **characterized by** the X-ray powder diffraction pattern shown in figure 7.

16. A crystalline polymorph of the compound according to claim 1, which is **characterized by** an IR absorption spectrum having characteristic peaks at approximately 3083 cm⁻¹, 2690 cm⁻¹, 2636 cm⁻¹, 2413 cm⁻¹, 2245 cm⁻¹, 1682 cm⁻¹, 1598 cm⁻¹, 1576 cm⁻¹, 1550 cm⁻¹, 1500 cm⁻¹, 1335 cm⁻¹, 1291 cm⁻¹, 1235 cm⁻¹, 1179 cm⁻¹, 1164 cm⁻¹, 1072 cm⁻¹, 1025 cm⁻¹, 978 cm⁻¹, 940 cm⁻¹, 816 cm⁻¹, 767 cm⁻¹, 707 cm⁻¹ and 639 cm⁻¹.

17. The crystalline polymorph of claim 16, **characterized by** the IR absorption spectrum shown in figure 8.

18. The compound according to claim 1, wherein at least 70% are a crystalline polymorph according to any of claims 2 to 17.

19. A process for the preparation of a compound according to any of claims 1 to 18, which process comprises reacting (2S)-1-{[1,1-Dimethyl-3-(4-pyridin-3-yl-imidazol-1-yl)-propylamino]-acetyl}-pyrrolidine-2-carbonitrile with fumaric acid.

20. A process according to claim 19, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from 1-propanol in the presence of water.

21. A process according to claim 19, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from 1-propanol in the absence of water.

22. A process according to claim 19, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from isopropanol.

23. A process according to claim 19, wherein the resulting compound (2S)-1-{[1,1-Dimethyl-3-(4-pyridin-3-yl-imidazol-1-yl)-propylamino]-acetyl}-pyrrolidine-2-carbonitrile fumarate is crystallized from THF.

24. Pharmaceutical compositions comprising a compound or crystalline polymorph according to any of claims 1 to 18 and a pharmaceutically acceptable carrier and/or adjuvant.

25. A compound or crystalline polymorph according to any of claims 1 to 18 for use as therapeutic active substance.

26. A compound or crystalline polymorph according to any of claims 1 to 18 for use as therapeutic active substance for the treatment and/or prophylaxis of diseases which are associated with DPP-IV.

27. The use of a compound or crystalline polymorph according to any of claims 1 to 18 for the preparation of medicaments for treatment and/or prophylaxis of diseases which are associated with DPP-IV.

28. The use of a compound or crystalline polymorph according to any of claims 1 to 18 for the preparation of medicaments for the treatment and/or prophylaxis of diabetes, non-insulin-dependent diabetes mellitus, impaired glucose tolerance, bowl disease, colitis ulcerosa, morbus crohn, obesity, and/or metabolic syndrome.

## Patentansprüche

1. Die Verbindung (2S)-1-{[1,1-Dimethyl-3-(4-pyridin-3-yl-imidazol-1-yl)-propylamino]-acetyl}-pyrrolidin-2-carbonitrilfumarat.

2. Kristalline polymorphe Form der Verbindung nach Anspruch 1, die durch ein Pulverdiffraktionsmuster mit charakteristischen Peaks, ausgedrückt in Grad 2-Theta bei ungefähr
| Grad 2-Theta |
|---|
| 9,0 |
| 10,8 |
| 14,1 |
| 19,6 |
| 21,6 |
**gekennzeichnet** ist.

3. Kristalline polymorphe Form nach Anspruch 2, **gekennzeichnet durch** das in Figur 1 gezeigte Pulverdiffraktionsmuster.

4. Kristalline polymorphe Form der Verbindung nach Anspruch 1, die durch ein IR-Absorptionsspektrum mit charakteristischen Peaks bei ungefähr 3382 cm⁻¹, 3128 cm⁻¹, 2654 cm⁻¹, 2451 cm⁻¹, 1714 cm⁻¹, 1662 cm⁻¹, 1601 cm⁻¹, 1266 cm⁻¹, 1202 cm⁻¹, 1162 cm⁻¹, 1074 cm⁻¹, 1031 cm⁻¹, 995 cm⁻¹, 976 cm⁻¹, 944 cm⁻¹, 915 cm⁻¹, 839 cm⁻¹, 817 cm⁻¹, 760 cm⁻¹, 710 cm⁻¹ und 637 cm⁻¹ **gekennzeichnet** ist.

5. Kristalline polymorphe Form nach Anspruch 4, **gekennzeichnet durch** das in Figur 2 gezeigte IR-Absorptionsspektrum.

6. Kristalline polymorphe Form der Verbindung nach Anspruch 1, die durch ein Pulverdiffraktionsmuster mit charakteristischen Peaks, ausgedrückt in Grad 2-Theta bei ungefähr
| Grad 2-Theta |
|---|
| 10,3 |
| 16,0 |
| 17,3 |
| 19,0 |
| 21,3 |
**gekennzeichnet** ist.

7. Kristalline polymorphe Form nach Anspruch 6, **gekennzeichnet durch** das in Figur 3 gezeigte Pulverdiffraktionsmuster.

8. Kristalline polymorphe Form der Verbindung nach Anspruch 1, die durch ein IR-Absorptionsspektrum mit charakteristischen Peaks bei ungefähr 3345 cm⁻¹, 3090 cm⁻¹, 2641 cm⁻¹, 2408 cm⁻¹, 2130 cm⁻¹, 1676 cm⁻¹, 1595 cm⁻¹, 1335 cm⁻¹, 1231 cm⁻¹, 1162 cm⁻¹, 1072 cm⁻¹, 1024 cm⁻¹, 976 cm⁻¹, 943 cm⁻¹, 819 cm⁻¹, 792 cm⁻¹, 709 cm⁻¹ und 631 cm⁻¹ **gekennzeichnet** ist.

9. Kristalline polymorphe Form der Verbindung nach Anspruch 8, die durch das in Figur 4 gezeigte IR-Absorptionsspektrum **gekennzeichnet** ist.

10. Kristalline polymorphe Form der Verbindung nach Anspruch 1, die durch ein Pulverdiffraktionsmuster mit charakteristischen Peaks, ausgedrückt in Grad 2-Theta bei ungefähr
| Grad 2-Theta |
|---|
| 6,4 |
| 11,0 |
| 11,5 |
| 14,0 |
| 19,6 |
**gekennzeichnet** ist.

11. Kristalline polymorphe Form nach Anspruch 10, **gekennzeichnet durch** das in Figur 5 gezeigte Pulverdiffraktionsmuster.

12. Kristalline polymorph Form der Verbindung nach Anspruch 1, die durch ein IR-Absorptionsspektrum mit charakteristischen Peaks bei ungefähr 3310 cm⁻¹, 2658 cm⁻¹, 2418 cm⁻¹, 2245 cm⁻¹, 1677 cm⁻¹, 1600 cm⁻¹, 1573 cm⁻¹, 1334 cm⁻¹, 1216 cm⁻¹, 1164 cm⁻¹, 1075 cm⁻¹, 1021 cm⁻¹, 960 cm⁻¹, 941 cm⁻¹, 822 cm⁻¹, 790 cm⁻¹, 711 cm⁻¹ und 635 cm⁻¹ **gekennzeichnet** ist.

13. Kristalline polymorphe Form nach Anspruch 12, **gekennzeichnet durch** das in Figur 6 gezeigte IR-Absorptionsspektrum.

14. Kristalline polymorphe Form der Verbindung nach Anspruch 1, die durch ein Pulverdiffraktionsmuster mit charakteristischen Peaks, ausgedrückt in Grad 2-Theta bei ungefähr
| Grad 2-Theta |
|---|
| 7,1 |
| 10,7 |
| 14,4 |
| 15,6 |
| 17,6 |
**gekennzeichnet** ist.

15. Kristalline polymorphe Form nach Anspruch 14, **gekennzeichnet durch** das in Figur 7 gezeigte Pulverdiffraktionsmuster.

16. Kristalline polymorphe Form der Verbindung nach Anspruch 1, die durch ein IR-Absorptionsspektrum mit charakteristischen Peaks bei ungefähr 3083 cm⁻¹, 2690 cm⁻¹, 2636 cm⁻¹, 2413 cm⁻¹, 2245 cm⁻¹, 1682 cm⁻¹, 1598 cm⁻¹, 1576 cm⁻¹, 1550 cm⁻¹, 1500 cm⁻¹, 1335 cm⁻¹, 1291 cm⁻¹, 1235 cm⁻¹, 1179 cm⁻¹, 1164 cm⁻¹, 1072 cm⁻¹, 1025 cm⁻¹, 978 cm⁻¹, 940 cm⁻¹, 816 cm⁻¹, 767 cm⁻¹, 707 cm⁻¹ und 639 cm⁻¹ **gekennzeichnet** ist.

17. Kristalline polymorphe Form nach Anspruch 16, **gekennzeichnet durch** das in Figur 8 gezeigte IR-Absorptionsspektrum.

18. Verbindung nach Anspruch 1, wobei mindestens 70 % eine kristalline polymorphe Form nach einem der Ansprüche 2 bis 17 sind.

19. Verfahren zur Herstellung einer Verbindung nach einem der Ansprüche 1 bis 18, wobei das Verfahren das Umsetzen von (2S)-1-{[1,1-Dimethyl-3-(4-pyridin-3-yl-imidazol-1-yl)-propylamino]-acetyl}-pyrrolidin-2-carbonitril mit Fumarsäure umfasst.

20. Verfahren nach Anspruch 19, wobei die resultierende Verbindung (2S)-1-{[1,1-Dimethyl-3-(4-pyridin-3-yl-imidazol-1-yl)-propylamino]-acetyl}-pyrrolidin-2-carbonitrilfumarat aus 1-Propanol in Gegenwart von Wasser kristallisiert wird.

21. Verfahren nach Anspruch 19, wobei die resultierende Verbindung (2S)-1-{[1,1-Dimethyl-3-(4-pyridin-3-yl-imidazol-1-yl)-propylamino]-acetyl}-pyrrolidin-2-carbonitrilfumarat aus 1-Propanol in Abwesenheit von Wasser kristallisiert wird.

22. Verfahren nach Anspruch 19, wobei die resultierende Verbindung (2S)-1-{[1,1-Dimethyl-3-(4-pyridin-3-yl-imidazol-1-yl)-propylamino]-acetyl}-pyrrolidin-2-carbonitrilfumarat aus Isopropanol kristallisiert wird.

23. Verfahren nach Anspruch 19, wobei die resultierende Verbindung (2S)-1-{[1,1-Dimethyl-3-(4-pyridin-3-yl-imidazol-1-yl)-propylamino]-acetyl}-pyrrolidin-2-carbonitrilfumarat aus THF kristallisiert wird.

24. Pharmazeutische Zusammensetzungen, umfassend eine Verbindung oder eine kristalline polymorphe Form nach einem der Ansprüche 1 bis 18 und einen pharmazeutisch akzeptablen Träger und/oder ein pharmazeutisch akzeptables Adjuvans.

25. Verbindung oder kristalline polymorphe Form nach einem der Ansprüche 1 bis 18 zur Verwendung als therapeutisch aktive Substanz.

26. Verbindung oder kristalline polymorphe Form nach einem der Ansprüche 1 bis 18 zur Verwendung als therapeutisch aktive Substanz für die Behandlung und/oder Vorbeugung von Erkrankungen, wie mit DPP-IV in Verbindung stehen.

27. Verwendung einer Verbindung oder einer kristallinen polymorphen Form nach einem der Ansprüche 1 bis 18 zur Herstellung von Medikamenten für die Behandlung und/oder Vorbeugung von Erkrankungen, die mit DPP-IV in Verbindung stehen.

28. Verwendung einer Verbindung oder einer kristallinen polymorphen Form nach einem der Ansprüche 1 bis 18 zur Herstellung von Medikamenten für die Behandlung und/oder Vorbeugung von Diabetes, nicht-insulinpflichtigem Diabetes mellitus, gestörter Glucosetoleranz, Darmerkrankung, Colitis ulcerosa, Morbus Crohn, Fettleibigkeit und/oder Stoffwechselsyndrom.

## Revendications

1. Composé fumarate de (2S)-1-{[1,1-diméthyl-3-(4-pyridine-3-yl-imidazole-1-yl)-propylamino]-acétyl}-pyrrolidine-2-carbonitrile.

2. Forme polymorphe cristalline du composé suivant la revendication 1, qui est **caractérisée par** un diagramme de diffraction des rayons X sur poudre présentant des pics caractéristiques exprimés en degrés 2-thêta à approximativement :
| **Degrés 2-thêta** |
|---|
| 9,0 |
| 10,8 |
| 14,1 |
| 19,6 |
| 21,6 |

3. Forme polymorphe cristalline suivant la revendication 2, **caractérisée par** le diagramme de diffraction des rayons X sur poudre représenté sur la figure 1.

4. Forme polymorphe cristalline du composé suivant la revendication 1, qui est **caractérisée par** un spectre d'absorption IR présentant des pics caractéristiques à approximativement 3382 cm⁻¹, 3128 cm⁻¹, 2654 cm⁻¹, 2451 cm⁻¹, 1714 cm⁻¹, 1662 cm⁻¹, 1601 cm⁻¹, 1266 cm⁻¹, 1202 cm⁻¹, 1162 cm⁻¹, 1074 cm⁻¹, 1031 cm⁻¹, 995 cm⁻¹, 976 cm⁻¹, 944 cm⁻¹, 915 cm⁻¹, 839 cm⁻¹, 817 cm⁻¹, 760 cm⁻¹, 710 cm⁻¹ et 637 cm⁻¹.

5. Forme polymorphe cristalline suivant la revendication 4, **caractérisée par** le spectre d'absorption IR représenté sur la figure 2.

6. Forme polymorphe cristalline du composé suivant la revendication 1, qui est **caractérisée par** un diagramme de diffraction des rayons X sur poudre présentant des pics caractéristiques exprimés en degrés 2-thêta à approximativement :
| **Degrés 2-thêta** |
|---|
| 10,3 |
| 16,0 |
| 17,3 |
| 19,0 |
| 21,3 |

7. Forme polymorphe cristalline suivant la revendication 6, **caractérisée par** le diagramme de diffraction des rayons X sur poudre représenté sur 1a figure 3.

8. Forme polymorphe cristalline du composé suivant 1a revendication 1, qui est **caractérisée par** un spectre d'absorption IR présentant des pics caractéristiques à approximativement 3345 cm⁻¹, 3090 cm⁻¹, 2641 cm⁻¹, 2408 cm⁻¹, 2130 cm⁻¹, 1676 cm⁻¹, 1595 cm⁻¹, 1335 cm⁻¹, 1231 cm⁻¹, 1162 cm⁻¹, 1072 cm⁻¹, 1024 cm⁻¹, 976 cm⁻¹, 943 cm⁻¹, 819 cm⁻¹, 792 cm⁻¹, 709 cm⁻¹ et 631 cm⁻¹.

9. Forme polymorphe cristalline suivant la revendication 8, qui est **caractérisée par** le spectre d'absorption IR représenté sur la figure 4.

10. Forme polymorphe cristalline du composé suivant la revendication 1, qui est **caractérisée par** un diagramme de diffraction des rayons X sur poudre présentant des pics caractéristiques exprimés en degrés 2-thêta à approximativement :
| **Degrés 2-thêta** |
|---|
| 6,4 |
| 11,0 |
| 11,5 |
| 14,0 |
| 19,6 |

11. Forme polymorphe cristalline suivant la revendication 10, **caractérisée par** le diagramme de diffraction des rayons X sur poudre représenté sur la figure 5.

12. Forme polymorphe cristalline du composé suivant la revendication 1, qui est **caractérisée par** un spectre d'absorption IR présentant des pics caractéristiques à approximativement 3310 cm⁻¹, 2658 cm⁻¹, 2418 cm⁻¹, 2245 cm⁻¹, 1677 cm⁻¹, 1600 cm⁻¹, 1573 cm⁻¹, 1334 cm⁻¹, 1216 cm⁻¹, 164 cm⁻¹, 1 075 cm⁻¹, 1 021 cm⁻¹, 960 cm⁻¹, 941 cm⁻¹, 822 cm⁻¹, 790 cm⁻¹, 711 cm⁻¹ et 635 cm⁻¹.

13. Forme polymorphe cristalline suivant la revendication 12, **caractérisée par** le spectre d'absorption IR représenté sur la figure 6.

14. Forme polymorphe cristalline du composé suivant la revendication 1, qui est **caractérisée par** un diagramme de diffraction des rayons X sur poudre présentant des pics caractéristiques exprimés en degrés 2-thêta à approximativement :
| **Degrés 2-thêta** |
|---|
| 7,1 |
| 10,7 |
| 14,4 |
| 15,6 |
| 17,6 |

15. Forme polymorphe cristalline suivant la revendication 14, **caractérisée par** le diagramme de diffraction des rayons X sur poudre représenté sur la figure 7.

16. Forme polymorphe cristalline du composé suivant la revendication 1, qui est **caractérisée par** un spectre d'absorption IR présentant des pics caractéristiques à approximativement 3083 cm⁻¹, 2690 cm⁻¹, 2636 cm⁻¹, 2413 cm⁻¹, 2245 cm⁻¹, 1682 cm⁻¹, 1598 cm⁻¹, 1576 cm⁻¹, 1550 cm⁻¹, 1500 cm⁻¹, 1335 cm⁻¹, 1291 cm⁻¹, 1235 cm⁻¹, 1179 cm⁻¹, 1164 cm⁻¹, 1072 cm⁻¹, 1025 cm⁻¹, 978 cm⁻¹, 940 cm⁻¹, 816 cm⁻¹, 767 cm⁻¹, 707 cm⁻¹ et 639 cm⁻¹.

17. Forme polymorphe cristalline suivant la revendication 16, **caractérisée par** le spectre d'absorption IR représenté sur la figure 8.

18. Composé suivant la revendication 1, dans lequel une proportion d'au moins 70 % est constituée d'une forme polymorphe cristalline suivant l'une quelconque des revendications 2 à 17.

19. Procédé pour la préparation d'un composé suivant l'une quelconque des revendications 1 à 18, procédé qui comprend la réaction du (2S)-1-{[1,1-diméthyl-3-(4-pyridine-3-yl-imidazole-1-yl)-propylamino]-acétyl}-pyrrolidine-2-carbonitrile avec de l'acide fumarique.

20. Procédé suivant la revendication 19, dans lequel le composé fumarate de (2S)-1-{[1,1-diméthyl-3-(4-pyridine-3-yl-imidazole-1-yl)-propylamino]-acétyl}-pyrrolidine-2-carbonitrile résultant est cristallisé à partir de 1-propanol en présence d'eau.

21. Procédé suivant la revendication 19, dans lequel le composé fumarate de (2S)-1-{[1,1-diméthyl-3-(4-pyridine-3-yl-imidazole-1-yl)-propylamino]-acétyl}-pyrrolidine-2-carbonitrile résultant est cristallisé à partir de 1-propanol en l'absence d'eau.

22. Procédé suivant la revendication 19, dans lequel le composé fumarate de (2S)-1-{[1,1-diméthyl-3-(4-pyridine-3-yl-imidazole-1-yl)-propylamino]-acétyl}-pyrrolidine-2-carbonitrile résultant est cristallisé à partir d'isopropanol.

23. Procédé suivant la revendication 19, dans lequel le composé fumarate de (2S)-1-{[1,1-diméthyl-3-(4-pyridine-3-yl-imidazole-1-yl)-propylamino]-acétyl}-pyrrolidine-2-carbonitrile résultant est cristallisé à partir de THF.

24. Compositions pharmaceutiques comprenant un composé ou une forme polymorphe cristalline suivant l'une quelconque des revendications 1 à 18 et un support et/ou adjuvant pharmaceutiquement acceptable.

25. Composé ou forme polymorphe cristalline suivant l'une quelconque des revendications 1 à 18, destiné à être utilisé comme substance thérapeutique active.

26. Composé ou forme polymorphe cristalline suivant l'une quelconque des revendications 1 à 18, destiné à être utilisé comme substance thérapeutique active pour le traitement et/ou la prophylaxie de maladies qui sont associées à DPP-IV.

27. Utilisation d'un composé ou d'une forme polymorphe cristalline suivant l'une quelconque des revendications 1 à 18 pour la préparation de médicaments destinés au traitement et/ou à la prophylaxie de maladies qui sont associées à DPP-IV.

28. Utilisation d'un composé ou d'une forme polymorphe cristalline suivant l'une quelconque des revendications 1 à 18 pour la préparation de médicaments destinés au traitement et/ou à la prophylaxie du diabète, du diabète sucré non insulinodépendant, de la tolérance entravée au glucose, d'une maladie intestinale, de la colite ulcérative, de la maladie de Crohn, de l'obésité et/ou du syndrome métabolique.
